# EUROPEAN PATENT APPLICATION

(11) **EP 1 548 116 A1**
(43) Date of publication of application: **29.06.2005**
(21) Application number: 03748615.6
(22) Date of filing: 30.09.2003
(51) Int. Cl.: C12N 15/63

(54) **METHOD OF PRODUCING STABLE ISOTOPE-LABELED PROTEIN**

(30) Priority: 30.09.2002 JP 2002285254
(71) Applicant: Ajinomoto Co., Inc., Tokyo 104-0031 (JP)
(72) Inventor: SHIMBA, Nobuhisa, AJINOMOTO CO., INC., Kawasaki-shi, Kanagawa 210-8681 (JP); KIKUCHI, Yoshimi, AJINOMOTO CO., INC., Kawasaki-shi, Kanagawa 210-8681 (JP); YAMADA, Naoyuki, AJINOMOTO CO., INC., Kawasaki-shi, Kanagawa 210-8681 (JP); SUZUKI, Ei-ichiro, AJINOMOTO CO., INC., Kawasaki-shi, Kanagawa 210-8681 (JP)
(74) Representative: HOFFMANN - EITLE
(86) International application number: PCT/JP2003/012506
(87) International publication number: WO 2004/029254

(57) **Abstract**

The present invention provides a method of producing a labeled protein, which comprises culturing a coryneform bacterium or a bacterium belonging to *Bacillus* in a medium which contains a carbon source and/or a nitrogen source labeled with one or more stable isotope selected from the group of consisting ²H, ¹³C and ¹⁵N and/or which contains ²H₂O, allowing the protein to be secreted into the medium and recovering the protein, wherein a genetic construct encoding the protein is introduced into the coryneform bacterium the bacterium belonging to *Bacillus*.

## Description

### BACKGROUND OF THE INVENTION

The present invention relates to a method of producing a protein labeled with a stable isotope. Particularly, the present invention relates to a method of producing a protein labeled with a stable isotope using a coryneform bacterium or a bacterium belonging to the genus Bacillus, wherein a genetic construct containing a gene encoding a heterogenous protein is introduced into the bacterium.

Extensive studies have been carried out on structural analysis, interaction analysis, and ligand screening of protein using NMR. Structural analysis leads to ligand design and protein engineering based on coordinate information of protein, enabling development of creation of drugs or high functional enzymes. Furthermore, since NMR are not only utilized to study the interaction sites and the structural change associated with interaction in analysis of signaling mechanism or metabolism in cells. NMR can detect even a weak interaction and clarify the interaction sites, and the application of NMR to ligand screening is also in progress. Particularly, the genome information is disclosed and cDNAs are accumulated for various species recently, and therefore the project for exhaustive structural analysis of proteins encoded by these genes is also in progress as a part of post-genome analysis, where NMR also plays the important role.

When analyzing protein by using NMR, stable isotope labeling is often performed. Nuclear species detected by NMR with good sensitivity include ¹H, ¹³C, and ¹⁵N. However, their naturally occuring ratios are low except ¹H. Therefore, a method where the signal numbers from ¹H in protein are decreased by ²H labeling and signals are sharpened for easy analysis (negative labeling), and a method where substituting to ¹³C and ¹⁵N is performed for multi dimensional NMR measurement (positive labeling), have been used for NMR analysis.

Stable isotope labeling of protein is mainly performed by constructing the expression system using Escherichia coli, and then culturing a recombinant Escherichia coli in a medium supplemented with labeled compounds. The labeled compounds include ¹³C-labeled glucose, ¹⁵NH₄Cl, ¹³C-labeled glycerol, and various labeled amino acids (for example, Biochemistry 21, 6273. (1982), Biochemistry 29, 4659. (1990), Journal of Biomol NMR, 20, 71. (2001)). Although the system using Escherichia coli may be easily constructed and is widely used, a protein is accumulated as a granule in the bacterial cells, which makes the refolding or purification of the protein difficult in many cases. Moreover, since E.coli cannot be cultured at a high density, the yield of protein is low and as much as 1 - 10 L of medium is required to prepare an NMR sample. Further, the labeling indices by the stable isotopes are at most approximately 60% for ²H, 95% for ¹³C, and 90% for ¹⁵N (Journal of Biomol NMR, 20, 71 (2001)).

Methods of producing a stable isotope labeled protein using an expression system for yeast or animal cells are also used in addition to the Escherichia coli system. Although labeled proteins are expressed as an active form in these systems, the problem of refolding can be avoided, the variety of expressed is limited and the expression level is low. Moreover, it has a large problem of an increased cost because they consume a large amount of stable isotope compounds. There are some reports on secretion of stable isotope labeled proteins at a relatively high efficiency using methanol-assimilating yeast, Pichia pastoris, among these systems (for example, Journal of Biomol NMR, 20, 251. (2001), Journal of Biomol NMR, 17, 337. (2000)). Although the labeling efficiency is high in the method using Pichia pastoris, it was necessary to continuously supply the medium containing expensive stable isotope compounds to perform continuous culture at a 300 ml scale.

To obtain a stable isotope labeled protein with a high labeling index, a medium containing higher concentration of stable isotope compounds is required and the cost increases consequently. For example, to obtain approximately 8 mg of stable isotope labeled protein (¹³C-stable isotope labeling ratio of 98%), it was reported that the large amounts of stable isotope labeled compounds, namely, a total of 15 g of ¹³C-glucose and 50 g of ¹³C-methanol were required (Journal of Biomol NMR, 20, 251. (2001)). For ¹⁵N-labeling, even if a high labeling index was desired, there has been a problem in that the expression of the protein decreased when Pichia pastoris was used and the concentration of (¹⁵NH₄)₂SO₄ was increased up to 20 g/L. Thus, there were many problems for the secretory production of stable isotope labeled proteins using the yeast, Pichia pastoris, which has a relatively high expression capacity.

Additionally, although a method of producing a stable isotope labeled protein by a cell free system has been also reported (J. Biomol. NMR 2, 129. (1995)), the protein was synthesized as an aggregate and refolding of the protein was difficult in many cases.

On the other hand, the previous investigations on efficient secretion of a foreign protein using coryneform bacteria include: secretion of nuclease and lipase (US4965197, J. Bacteriol., 174, 1854-1861 (1992)) and secretion of protease such as subtilisin (Appl. Environ. Microbiol., 61, 1610-1613 (1995)) by *Corynebacterium glutamicum* (hereinafter, may be abbreviated as C. glutamicum), secretion of cell surface protein of coryneform bacteria (Japanese Patent Un-examined Published Application (JP Kokai) No. 6-502548), secretion of fibronectin binding protein using coryneform bacteria (Appl. Environ. Microbiol., 63, 4392-4400 (1997)), improvement of protein secretion using mutant protein secretion system (JP Kokai No. 11-169182). Recently, a high production level was obtained in an expression system of EGF (human epidermal growth factor) and MTG (microbial transglutaminase), which was established by using coryneform bacteria (WO 01/23591).

Gene manipulation technology of coryneform bacteria has also been developed in the systems using plasmid or phage, for example, establishment of the transformation method by protoplast (J. Bacteriol., 159, 306-311(1984); J. Bacteriol., 161, 463-467(1985)), development of various kinds of vectors (Agric. Biol. Chem., 48, 2901-2903(1984); J. Bacteriol., 159, 306-311(1984); J. Gen. Microbiol., 130, 2237-2246(1984); Gene, 47, 301-306(1986); Appl. Microbiol. Biotechnol., 31, 65-69(1989)), development of gene expression controlling method, (Bio/Technology, 6, 428-430 (1988)), and development of cosmid, (Gene, 39, and 281-286 (1985)). Moreover, there are also reports on gene cloning from coryneform bacteria (Nucleic Acids Res., 14, 10113-1011 (1986); J. Bacteriol., 167, 695-702(1986); Nucleic Acids Res., 15, 10598(1987); Nucleic Acids Res., 15, 3922(1987); Nucleic Acids Res., 16, 9859(1988); Agric. Biol. Chem., 52, 525-531 (1988); Mol. Microbiol., 2, 63-72(1988); Mol. Gen. Genet., 218, 330-339(1989); Gene, 77, 237-251(1989)).

There are also reports on transposable elements from coryneform bacteria (WO93/18151; EP0445385; JP Kokai No. 6-46867; Mol. Microbiol., 11, 739-746(1994); Mol. Microbiol., 14, 571-581(1994); Mol. Gen. Genet., 245, 397-405(1994); FEMS Microbiol. Lett.,126, 1-6(1995); JP Kokai No. 7-107976).

The transposable element is a DNA fragment that can transpose on a chromosome and is known to exist in organisms ranging widely from prokaryote to eukaryote. A transposon using a transposable element has been developed (WO 93/18151; JP Kokai No. 7-107976; Mol. Gen. Genet., 245, 397-405(1994); JP Kokai No. 9-70291), which allows expressing foreign genes by the transposon.

On the other hand, in the researches on efficient secretory production of a protein by culturing *Bacillus brevis* at a high density, a system has been established where a secretion vector was generated using the promoter of the gene of MW protein (middle wall protein (MWP)), a kind of major extracellular proteins of *Bacillus brevis* 47 (JP Kokai No. 60-58074, JP Kokai No. 62-201583) and the signal peptide region encoded by the gene, and *Bacillus brevis* 47 was used as a host. Additionally, secretion of α-amylase (JP Kokai No. 62-201583, J. Bacteriol. 169, 1239 (1987)) or pig pepsinogen (Nihon-nogeikagakukaishi, 61, 68 (1987)) was succeeded. Further, *Bacillus brevis* HPD31 strain (also known as *Bacillus brevis* H102), which extracellularly produces less protease among *Bacillus brevis*, was isolated and was used successfully as a host for high secretory production of heat-resistant α-amylase (Agric. Biol. Chem., 53, 2279-2280 (1989)), high secretory production of human epidermal growth factor (EGF) (Proc. Natl. Acad. Sci. USA, 86, 3589-3593 (1989), JP Kokai No. 4-278091), and secretory production of human interleukin-2 (Biosci. Biotechnol. Biochem., 61, 1858-1861 (1997)) and human interleukin-6 (Biosci. Biotechnol. Biochem. 64, 665-669 (2000)). In particular, extremely high secretion accumulation of 1.1 g/L was obtained for EGF.

However, it has not yet been reported on secretion of stable isotope labeled proteins using coryneform bacteria or Bacillus bacteria (bacteria belonging to the genus Bacillus).

### SUMMARY OF THE INVENTION

An object of the present invention is to provide at a low cost a stable isotope labeled protein having a high labeling index, high yield, and high purity.

The inventors have focused attention on the fact that coryneform bacteria and Bacillus bacteria such as *Corynebacterium glutamicum* and *Bacillus brevis* naturally produce extremely small amount of extracellular protein, which enables simplification or elimination of the purification process after secretory production of foreign proteins, and that coryneform bacteria proliferate well in a simple medium containing sugar, ammonia, inorganic salts, and the like, which is advantageous in respect of the cost needed for a medium containing stable isotope labels and for the setting of the culture conditions, as well as for the productivity. The inventors have also focused attention on the point that coryneform bacteria and Bacillus bacteria such as *Corynebacterium glutamicum* and *Bacillus brevis* can be cultured at a high bacterium density and that a high yield can be expected, thereby enabling to decrease the consumption of expensive stable isotope compounds. Then, high-density culture was studied in the medium containing glucose, ammonium salts, ²H₂O, and the like which are the supply source of stable isotopes. As a result, the inventors have succeeded in secretory production of stable isotope labeled proteins with extremely high labeling indices even in the medium in which these bacteria exist at a high concentration. More specifically, it was found that the target proteins could be labeled with ¹³C and/or ¹⁵N at a high labeling index by adding ¹³C-labeled glucose and/or ¹⁵N-labeled ammonium chloride salt into the medium and utilizing them as a carbon or a nitrogen source, which lead to the present invention. It was also found that the ²H labeled protein having an extremely high labeling index could be secreted by culturing the bacteria in 90% ²H₂O.

Accordingly, the present invention provides a method of producing a stable isotope labeled protein which comprises culturing coryneform bacteria or Bacillus bacteria into which a genetic construct containing a gene encoding a foreign protein has been introduced, in a medium containing a carbon source and/or a nitrogen source labeled with at least one stable isotopes selected from ²H, ¹³C, and ¹⁵N and/or containing ²H₂O, allowing the secretion of the foreign protein into the medium and collecting the protein.

In particular, the present invention also provides a method of producing a stable isotope labeled protein wherein coryneform bacterium is *Corynebacterium glutamicum* in the above-mentioned method.

Furthermore, the present invention also provides a method of producing a stable isotope labeled protein wherein Bacillus bacterium is *Bacillus brevis* in the above-mentioned method.

Further, the present invention also provides a method of producing a stable isotope labeled protein wherein the carbon source is selected from a group consisting of glucose, glycerol, and sucrose and the nitrogen source is ammonium chloride salt or ammonium sulfate salt.

The term "(is) secreted" as used herein in the context with a protein and/or a peptide means that a protein molecule or a peptide molecule is transported toward outside of a bacterium (extracellular). The term also includes the cases in which the protein or peptide molecule is finally in the completely free form in a medium as well as the cases in which only a part of the molecule exists outside of the bacterium or the molecule remains on the surface of the bacterium.

### BRIEF DESCRIPTION OF THE DRAWINGS

Figure 1 shows a schematic drawing illustrating the advantage of using labeled compounds at a high concentration.
Figure 2 indicates the relationship of the growth of bacteria and the amount of pre-culture added into the main culture.
Figure 3 shows the MS spectra of unlabeled EFG, ¹⁵N-labeled EFG and ¹³C, ¹⁵N-labeled EGF.
Figure 4 shows the HSQC spectra of ¹⁵N-labeled EGF and MTG which were secreted into the medium.
Figure 5 shows the HSQC spectra of MTGs which were labeled by using (a) Coryneform bacteria and (b) E.coli.

### DESCRIPTION OF THE PREFERRED EMBODIMENTS

As described above, Escherichia coli has been conventionally used for producing stable isotope labeled proteins. However, refolding process is required to obtain a protein in its active form and trials and errors are required to establish correct refolding. Although a method of secreting the produced protein as its active form is also known, the yield is still low even if an yeast, which is known to exhibit high efficiency, was used for the production and a large amount of expensive stable isotope labeled compounds are consumed. The present invention may overcome such problems. Several particular embodiments of the present invention will be described below.

In the present invention, it is preferable to use a microorganism such as coryneform bacteria or Bacillus bacteria, which can be cultured at a high density and which can produce and secrete a foreign protein. The advantage of using such microorganisms, which can be cultured at a high density and which can produce and secrete a foreign protein, particularly coryneform bacteria and Bacillus bacteria, is that the amount of unintended proteins which may be extracellularly secreted is extremely small and that the purification process can be simplified after the production of the intended protein as compared with yeast or insect cells which has previously been used for secretory expression of proteins.

Additionally, for example, when insect cells are used, it is necessary to grow them in the medium enriched with sugars and amino acids, while it is possible to use only sugars, ammonium salts, and the like as a carbon source and a nitrogen source when such microorganisms are used, which is preferable for introduction of stable isotope atoms. Furthermore, when such microorganisms are used, sufficient amount of sample proteins for NMR analysis may be obtained by a small-scale batch culture, because the expression by the microorganisms is high and they can be cultured at a high density. Therefore, stable isotope labeled protein can be produced with a simple equipment, and the consumption of expensive stable isotope compounds may be significantly reduced as compared with yeast, which improves the economical efficiency and productivity as compared with the conventional methods. Furthermore, since such microorganisms can be cultured in the medium containing stable isotope source (for example, sugar, ammonium salts) at a high concentration, the concentrations of the stable isotopes in the medium can be highly increased and a stable isotope labeled protein having a labeling index as extremely high as 99% or more can be produced and secreted.

Coryneform bacteria as used herein refers to an aerobic gram-positive bacillus as described in Bergey's Manual of Determinative Bacteriology eighth edition, p599 (1974), coryneform bacterium, which were conventionally classified as Brevibacterium, and which presently includes the microorganisms integrated into Corynebacterium (Int. J. Syst. Bacteriol., 41, 255 (1981)) and also includes Brevibacterium which is closely related with Corynebacterium.

The coryneform bacteria which can be used in the present invention include L-glutamate producing bacteria including, the wild strains, e.g. *Brevibacterium saccharolyticum* ATCC14066, *Brevibacterium immariophilum* ATCC14068, *Brevibacterium lactofermentum* (*Corynebacterium glutamicum*) ATCC13869, *Brevibacterium roseum* ATCC13825, *Brevibacterium flavum* ATCC14067, (*Corynebacterium glutamicum*), *Corynebacterium acetoacidophilum* ATCC13870, *Corynebacterium glutamicum* ATCC13032, *Corynebacterium lilium* (*Corynebacterium glutamicum*) ATCC15990, *Brevibacterium ammoniagenes,* (*Corynebacterium ammoniagenes*) ATCC6871, mutants derived from these wild strains such as the mutants which lost glutamate producing capacity, mutants producing amino acid such as lysine, and mutants producing other substances such as nucleic acid, for example, inosine.

Bacillus bacteria which can be used in the present invention include *Bacillus brevis. Bacillus brevis* is an aerobic gram-positive bacillus and the followings are utilized: for example, *Bacillus brevis* 47 (JP Kokai No. S60-58074, JP Kokai No. S62-201583) and *Bacillus brevis* H102 (JP Kokai No. S63-56277). Preferably, *Bacillus brevis* H102 is used. These mutants of these strains are also included in the "Bacillus bacteria" as used herein.

Generally, the genetic construct used for the present invention has a promoter, a nucleic acid fragment that encodes a suitable signal peptide sequence and the interested protein, and control sequences (for example, an operator, terminator and the like) required to express the gene of the interested protein in a host at a suitable position such that they can function.

Generally, the genetic construct used for the present invention has, for example, a promoter, a nucleic acid fragment that contains a suitable signal peptide and a control sequence (for example, a promoter or a terminator) which is required to express the target protein gene in coryneform bacteria or Bacillus bacteria at a suitable position. The vectors which can be used in the construct are not particularly limited. They may be those which can function in a host such as coryneform bacteria or Bacillus bacteria including a vector which can autonomously replicate extrachromosomaly such as a plasmid, or a vector which can be integrated into the bacterial chromosome. Depending on a host, plasmids from coryneform bacteria or Bacillus bacteria is particularly preferable. These include pHM1519 (Agric, Biol. Chem., 48, 2901-2903(1984)), pAM330 (Agric. Biol. Chem., 48, 2901-2903(1984)), pHT926 (Japanese Patent Application No. 4-216605), and the plasmid having a drug resistant gene that is produced by improving them. When using *Bacillus brevis* as a host, pUB110 (J. Bacteriol., 134,318-329 (1978)) which has also been used for *Bacillus subtilis.*

Transposon can also be used. When transposon is used, the interested gene is introduced into a chromosome by homologous recombination or by its transpositional capacity. The usable promoters, SDs, and signal peptide encoding genes are not particularly limited, and any can be used as long as it can function in the host.

The promoters which can be used for the present invention are not particularly limited. Generally, any promoters which can function in a host microorganism may be used. Additionally, the promoters may be heterologous promoters such as promoters from E. coli including *tac* promoter. Among them, a strong promoter such as tac promoter is more preferable.

The promoters from coryneform bacteria include the promoters of the gene for cell surface proteins, PS1, PS2, and SlpA, glutamic acid dehydrogenase gene of various kinds of amino acid biosynthesis systems such as glutamic acid biosynthesis system, glutamine synthetase gene of glutamine synthesis system, aspartokinase gene of lysine biosynthesis system, homoserine dehydrogenase gene of threonine biosynthesis system, acetohydroxy acid synthase gene of isoleucine and valine biosynthesis system, 2-isopropylmalic acid synthase gene of leucine biosynthesis system, glutamate kinase gene of proline and arginine biosynthesis system, phosphoribosyl-ATP pyrophosphorylase gene of histidine biosynthesis system, deoxyarabinoheptulosonate phosphoric acid (DAHP) synthase gene of aromatic amino acid biosynthesis systems such as tryptophan, tyrosine, and phenylalanine, phosphoribosyl pyrophosphate (PRPP) amidotransferase gene of nucleic acid biosynthesis system such as inosinic acid and guanylic acid, inosinic acid dehydrogenase gene, and guanylic acid synthase gene. For example, when Bacillus bacteria, particularly *Bacillus brevis,* are used as hosts, a promoter, SD, and a signal peptide encoding the gene from *Bacillus brevis* 47 or H102 can be used (J. Bacteriol., 170,935-945 (1998), J. Bacteriol., 172, 1312-1320 (1990)).

The proteins which may be labeled in the present invention are not particularly limited. When a protein which is not naturally secreted is labeled according to the present invention, the protein may be labeled after modifying the protein into a secretory form, for example, by connecting it to a suitable signal peptide.

The proteins which can be labeled with stable isotopes according to the present invention are not limited and include substantially all the proteins from animals, plants, or microorganisms. For example, the following proteins may be labeled with stable isotopes according to the present invention: human plasma components such as albumin, immunoglobulin, and blood coagulation factor; enzymes such as protease and transferase; hormones such as growth hormone and erythropoietin; cell growth factors or cell inhibition factors; immunoregulatory factors such as cell differentiation, induction, and stimulation; biologically active proteins produced by cells such as monokine, cytokine and lymphokine. In the following examples, the embodiments are shown where microbial transglutaminase (MTG) and human epidermal growth factor (EGF) were labeled with stable isotopes according to the method of the present invention.

When a protein that is to be labeled according to the present invention is naturally expressed as a prepropeptide, the gene encoding the proprotein may be also used. The pro-structure part of the protein obtained by expressing the gene may be cleaved by suitable methods, for example using proteases, aminopeptidases or endopeptidases which cleave the protein at an appropriate position, or more specific protease may also be used.

In the present invention, signal peptides which can be used to secrete proteins are preferably the signal peptides of the secretory proteins from the host, a coryneform bacterium or a bacillus. For example, when coryneform bacteria are used, signal peptides of the cell surface protein of coryneform bacteria are preferable. The cell surface proteins of coryneform bacteria include PS1 and PS2 from C. glutamicum (JP Kokai No. 6-502548), and SlpA derived from C. ammoniagenes (JP Kokai No. 10-108675). US Patent No. 4965197 describes that DNase from coryneform bacteria has also a signal peptide. Such a signal peptide can also be used. When Bacillus bacteria are used, for example, the signal peptide of MW protein or OW protein, which is one of the major extracellular proteins of *Bacillus brevis* 47 (JP Kokai No. 60-58074, JP Kokai No. 62-201583) or the signal peptide of the α-amylase of *Bacillus subtilis* may be used.

A signal sequence will be cleaved by a signal peptidase when the translated product is extracellularly secreted from the bacterium. Although the natural form of a gene encoding a signal peptide can be used, it can be modified so that the gene has the optimal codon depending on the codon usage of the host used. When such a signal peptide is used, a gene encoding the interested protein is connected to the 3'-end of a gene encoding the signal peptide and may be positioned such that the above-mentioned promoter can control the gene expression.

In the present invention, carbohydrate such as glucose and sucrose, organic acids such as acetic acid, alcohols such glycerol, amino acids, or the like can be used as carbon sources in the medium. Among them, glucose, sucrose, and glycerol, which are relatively inexpensive, are preferable. As nitrogen sources, ammonia gas, aqueous ammonia, ammonium salts, amino acids or the like can be used. In particular, ammonium chloride and ammonium sulfate are preferable, which may be easily available and which is easy to be handled. For labeling with stable isotopes, it is preferable that the carbon or nitrogen source species are less. Coryneform bacteria used for the present invention are preferable, because they utilize glucose and ammonium salt alone as a carbon and a nitrogen sources, respectively. If glucose is labeled with ¹³C, ¹³C-labelling may be achieved and if ammonium salts are labeled with ¹⁵N, ¹⁵N-labeling may be achieved.

The concentration of labeled carbon sources in the medium is preferably 5 g/L - 150 g/L (approximately 27 mM - 0.81 M) calculated as ¹³C-labeled glucose concentration, more preferably, 20 g/L - 60 g/L (approximately 107 mM - 322 mM), and most preferably, 30 g/L - 50 g/L (approximately 160 mM - 269 mM). When ammonium salts are used as nitrogen sources for labeling, the ¹⁵NH₄⁺ concentration is preferably 30 mM - 1 M, more preferably, 50 mM - 600 mM, and most preferably, 200 mM - 400 mM.

The growth of bacteria will be improved when they are pre-cultured before culturing the bacterium in the medium containing labeled compounds (main culture) as shown in Example 1. The lesser amount of the preculture added to the main culture is preferable, because the labeling index increases. However, if the amount of added preculture medium is too small, it will have an adverse influence on growth of the bacterium. Generally, 0.5% or more of the amount of the preculture medium is preferable to be added into the main culture for the good growth of the bacterium, and it is preferable that the concentration is approximately 10% of at most to increase the labeling indices, as described below. In one embodiment of the present invention, it is shown that the optimal concentration of the preculture medium added to the main culture is approximately 2.5% (v/v).

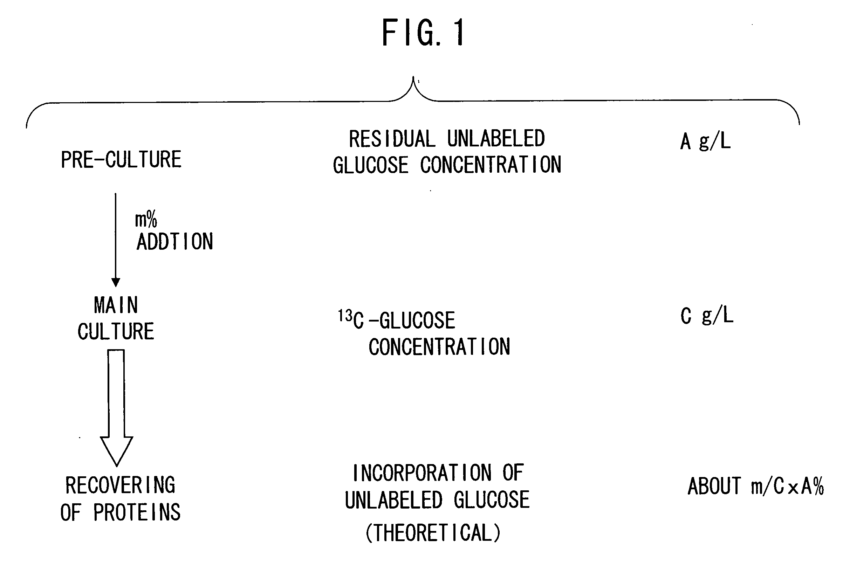

The high concentrations of the labeled compounds contained in the main culture may improve the labeling indices of proteins (see Fig. 1). When it is assumed that the preculture medium in which A g/L of non-labeled glucose remains is added by 2.5% to the main culture and the ¹³C-glucose concentration of the main culture is assumed to be 2 g/L as in a conventional case where Escherichia coli is cultured to obtain the labeled protein, 1.25 x A% of the non-labeled glucose contained in the preincubation medium will be incorporated. On the other hand, when the high concentration of ¹³C-glucose, for example, 60 g/L, is used as in the present invention, only 0.042 x A% of the non-label glucose is incorporated and thus a high labeling ratio can be attained. It is also the same for ²H or ¹⁵N. Thus, although the concentration of the preculture medium added to the main culture is not necessarily limited strictly, to obtain a high labeling index while maintaining a good growth of a bacterium, the concentration is preferably 0.5% - 15% (v/v), more preferably 1% - 10% (v/v) and 1.5% - 5% (v/v). The concentrations of the stable isotope labeled carbon and/or the nitrogen sources in the medium can also be varied depending on the concentration of the preculture medium added to the main culture (see Fig. 1).

When Escherichia coli or the like is used, excessively high concentration of glucose or salts having stable isotope labels in the medium may cause a trouble in growth of the bacteria, but such a high concentration does not raise a problem for Bacillus bacteria, particularly *Bacillus brevis* or coryneform bacteria. Even if glucose and ammonium salt(s) are used at a high concentration, lesser culture amounts of these microorganisms are required and cost will not increase.

Thus, in one embodiment of the present invention (for example, Example 3), stable isotope labeled protein having a labeling index as high as very close to 100% can be obtained by culturing the bacteria in the medium containing labeled compounds at a high concentration. In Example 3, a flask culture on a 20 ml scale is shown. Thus, since the interested protein can be obtained at a high yield by a small volume- and high density-culture, approximately 2 mg - 9 mg of a stable isotope labeled protein can be produced and secreted by using a small amount of stable isotope labeled compounds, for example, 0.8 g - 1.2 g stable isotope labeled glucose or 0.35 g - 0.6 g of stable isotope labeled ammonium chloride salts. Therefore, it is possible to produce the interested labeled protein at a low cost as compared to the case where yeast, Pichia pastoris consumes 15 g of glycerol and 50 g of methanol to produce 8 mg of a protein (Journal of Biomol NMR, 20, 251 (2001)).

Moreover, it is possible to produce ²H labeled proteins by a culture in a medium containing ²H₂O solvent together with ²H-labeled glucose, succinic acid, or the like. In another embodiment of the present invention, without using expensive ²H labeled glucose or amino acid, the interested stable isotope labeled protein can be produced and secreted at a high labeling index of 90% by a culture in a medium prepared with ²H₂O (Example 4). Furthermore, a protein labeled with two or three species of stable isotopes can be obtained by adding a combination of the compounds labeled with these stable isotopes into the medium.

Since a medium containing ²H₂O solvent is needed in producing the ²H labeled protein, a large amount of ²H₂O is required in the conventional method. However, according to the present invention, since high-density culture is possible, the amount of the medium containing ²H₂O may be small. Regarding the labeling index, extremely high ²H-labeling can be achieved as compared with Escherichia coli where the index was 60% (Journal of Biomol NMR, 20, 71 (2001)). Although the exact labeling index for yeast has not been described, a large amount of ²H₂O is required due to a continuous culture.

In the present invention, amino acids and vitamins may be added to the medium to promote the growth of bacteria and the secretion of proteins. In addition to the carbon source and the nitrogen source, calcium ions, magnesium ions, phosphoric acid ions, potassium ions, iron ions, and the like may be used as inorganic ions if needed. The culture may be performed under aerobic conditions and in the range of temperature and pH suitable for growth of a host, for example, pH of 5.0 - 8.5 and 15 °C - 37 °C for approximately 1 - 7 days. A large amount of the interested protein can be produced in the bacterial cells and can be extracellularly secreted efficiently by culturing transformants under such conditions. When the bacterium to be used has an amino acid(s) requirement, the amino acid(s) can also be added to improve the growth of the bacterium or to increase the protein expression. In this case, it is preferable to use the amino acid(s) labeled with the same stable isotope(s) as that with which other substances in the medium are labeled.

The stable isotope labeled protein secreted in the medium according to the present invention can be isolated and purified from the medium by a well-known method after culturing. For example, after removing the bacteria by centrifugation, isolation and purification can be carried out according to the known suitable procedures such as salting out, ethanol precipitation, ultrafiltration, gel filtration chromatography, ion exchange column chromatography, affinity chromatography, middle or high pressure liquid chromatography, reversed phase chromatography, hydrophobic chromatography, or combinations of these methods. Moreover, NMR spectrum and MS spectrum of the stable isotope labeled protein thus obtained can be determined according to the well-known procedures. In particular, according to the present invention, contaminated proteins hardly exists in the medium after the culture, so that NMR spectrum can be determined by merely removing the bacterium by centrifugal separation and the like, and then eliminating low molecular compounds by dialysis, followed by a concentration procedure of the protein through ultrafiltration.

Although the present invention will be explained more specifically by the following examples, these examples should not be construed to limit the present invention in any way.

### Examples

### Reference Example 1: Perparation of cell surface protein (PS2) gene -disrupted strain from Corynebacterium glutamicum AJ 12036

The streptomycin (Sm) resistant strain AJ12036 had been bred from *Corynebacterium glutamicum* ATCC13869 and AJ12036 had been used as a host for genetic recombination in *Corynebacterium glutamicum* (U.S. Patent No. 4,822,738). *Corynebacterium glutamicum* (formerly Brevibacterium lactofermentum) AJ12036 was deposited in National Institute of Bioscience and Human-Technology, Agency of Industrial Science and Technology, Ministry of International Trade and Industry (currently, International Patent Organism Depository, National Institute of Advanced Industrial Science and Technology, Tukuba Central 6, 1-1, Higashi 1-Chome Tukuba-shi, Ibaraki-ken, 305-8566 Japan) on March 26, 1984 as FERM P-7559 and was transferred to International Deposit under Budapest Treaty on March 13, 1985 as FERM BP-734.

Since AJ 12036 was confirmed to slightly secrete the cell surface protein (PS2) into the medium, it had been expected that the efficiency of protein secretion could be further improved by gene disruption so that AJ12036 would not produce PS2 at all. Therefore, a complete PS2 gene-disrupted strain was generated by homologous recombination, as described below.

The following primers were synthesis based on the chromosomal DNA of *Corynebacterium glutamicum* ATCC13869, which was prepare according to the method of Saito & Miura (Biochim. Biopys. Acta., 72 619 (1963)), and PCR was conducted by using the combination of SEQ ID NOs:1 and 2 and the combination of SEQ ID NOs: 3 and 4. The gene sequence of PS2 of *Corynebacterium glutamicum* ATCC13869 has been described in U.S. Patent No. 5,547,864.

Each amplified fragment was used for crossover-PCR with the primers of SEQ ID NOs:1 and 3 to amplify ΔPS2 fragment in which the promoter region and the N-terminal region of the coding region of PS2 gene were deleted. The fragment was cloned into the Smal site of pUC19 to generate pUΔPS2. pUΔPS2 was digested with Kpnl and Xbal to excise ΔPS2 fragment which in turn inserted into the Kpnl-Xbal site of the temperature sensitive plasmid pHS4 (U.S. Patent No. 5,616,480) derived from pHM1519 to pHS ΔPS2. Escherichia coli Aj12570 transformed with plasmid pHS4 was deposited in National Institute of Bioscience and Human-Technology, Agency of Industrial Science and Technology, Ministry of International Trade and Industry (currently, International Patent Organism Depository, National Institute of Advanced Industrial Science and Technology, Tukuba Central 6, 1-1, Higashi 1-Chome Tukuba-shi, Ibaraki-ken, 305-8566 Japan) on October 11, 1990 as FERM P-11762 and was transferred to the International Deposit under Budapest Treaty on August 26, 1991 as FERM BP-3523. (SEQ ID NO:1)     5'-act ggg agg cta tct cca tt-3' (SEQ ID NO:2)     5'-atc gat ctg atc acg tta ca-3' (SEQ ID NO:3)     5'-tgt aac gtg atc aga tcg att cac tgg tcg aca ccg ttg a-3' (SEQ ID NO:4)    5'-acg gaa gct acc ttc gag gt-3' Sequence listing free text
SEQ ID NOs:1-4 PCR primer

pHS ΔPS2 was then introduced into AJ12036 strain by electroporation and a gene-disrupted strain where PS2 was completely disrupted was obtained through homologous recombination described in Japanese Patent No. 2763054. The resulting strain was designated as YDK010 strain.

### Reference Example 2: Construction of hEGF gene having the signal sequence of the cell surface protein from C. glutamicum ATCC13869

PS2 gene, which is the cell surface protein of C. glutamicum has been determined (Mol. Microbiol., 9, 97-109 (1933)). The primers indicated in SEQ ID NOs:5 and 6 are synthesized and used for amplifying the 5'-upstream region and the region encoding N-terminal 44 residues of the protein corresponding to PS2 including its signal sequence from the chromosomal DNA of C. glutamicum ATCC13869 prepared according to the method of Saito and Miura (Biochem. Biophys. Act., 72, 619 (1963)). Pyrobest DNA polymerase (Takara Shuzo) was used for PCR reaction in accordance with the reaction condition of the attached protocol. The primer indicated as SEQ ID NO:5 included the recognition site of Kpnl at the 5'-end , which is required for insertion into a plasmid. 
(SEQ ID NO:5)
5'-GCTCGGTACCCAAATTCCTGTGAATTAGCTGATTTAG-3'
 (SEQ ID NO:6)    5'-GTTGAAGCCGTTGTTGATGTTGAA-3' Sequence listing free text
SEQ ID NOs:5 and 6: PCR primers

The primers indicated in SEQ ID NOs:7 and 8 were synthesized, which were used for amplifying the region encoding hEGF by PCR method from the plasmid pT13SΔhIL2-KS-hEGF(H3) (JP Kokai No.64-4538) which contained hEGF gene sequence. E.coli Aj12354 transformed with the plasmid pT13SΔhIL2-KS-hEGF(H3) containing the gene was deposited in the National Institute of Bioscience and Human-Technology, Agency of Industrial Science and Technology, Ministry of International Trade and Industry (currently, International Patent Organism Depository, National Institute of Advanced Industrial Science and Technology, Tukuba Central 6, 1-1, Higashi 1-Chome Tukuba-shi, Ibaraki-ken, 305-8566 Japan) on November 20, 1987 as FERM P-9719 and transferred to the International Deposit under Budapest Treaty on March 18, 2002 as FERM BP-7966.

The primer indicated in SEQ ID NO:7 contained the gene sequence C-terminal amino acids of the PS2 signal sequence for constructing a fusion gene containing the 5'-region and the region encoding the N-terminal 44 amino acids residues of the protein corresponding to PS2. 
(SEQ ID NO:7)
5'-AACATCAACAACGGCTTCAACAATTCCGATTCTGAGTGCCCT-3'
 
(SEQ ID NO:8)
5’-CGGCCACGATGCGTCCGGCG-3’
 Sequence listing free text
SEQ ID NOs:7, 8: PCR primer

One µl of PCR reaction solution of amplified region containing 5'-upstream region and N-terminal 44 amino acid coding region and 1 µl of PCR reaction solution of amplified hEGF gene region were then mixed and used as the template for crossover PCR with SEQ ID NOs:5 and 8 to amplify the fusion gene where 5'-upstream region and the region encoding the N-terminal 44 amino acid residues which are linked to hEGF. The amplified fragment of about 0.9kb was detected by agarose gel electrophoresis. The fragment was recovered from the agarose gel by using EASY TRAP Ver.2 (Takara Shuzo). The recovered DNA was digested with Kpnl and BamHl (Takara Shuzo) and purified by using DNA Clean-UP system (Promega), and inserted into the Kpnl-BamHl site of plasmid pPK4 described in JP Kokai No.9-322774 to obtain pPKEGF. The nucleotide sequence of the inserted fragment was determined with Dye Terminator Cycle Sequence Kit (PE Applied Biosystems) and DNA Sequencer 377 (PE Applied Biosystems) to confirm that the expected fusion gene was constructed.

### Reference Example 3: Generation of hEGF producing strain

C. glutamicum YDK0101 generated in Reference Example 1 was transformed with the hEGF expression plasmid pPDEGF generated in Reference Example 2 to obtain Kanamycin resistant strains. Each of the resulting strains was cultured at 30°C for 3 days in a MMTG liquid medium (60g of glucose, 0.4g of magnesium sulfate heptahydrate, 30g of ammonium sulfate, 1g of potassium dihydrogen phosphate, 0.01g of iron sulfate heptahydrate, 0.01g of manganese sulfate pentahydrate, 450µg of thiamine hydrochloride, 450µg of biotin, 0.15g of DL-methionine, 50g of calcium carbonate, adjusted to 1 L with water and pH 7.5) containing 25mg/l of kanamycin. The bacterial cell was removed by centrifugation to obtain a supernatant and 10 µl of the supernatant was subjected to SDS-PAGE. The commercially available hEGF (PEPRO TECHEC LTD) was simultaneously electrophoresis as a standard and Coomassie Brilliant Blue staining was conducted, which resulted in the detection of a band at the location of the same mobility as the standard. There were little other proteins found in the culture supernatant.

### Reference Example 4: Secretory Production of MTG using Corynebacterium glutamicum YDK0101

The secretory expression plasmid of transglutaminase having modified pro-structure, pPKSPTG11 (WO 01/23591), and the serine protease expression plasmid pVSS1 were introduced into YDK010 strain which were generated in Reference Example 1 to obtain transformants.

The bacterial stains which could grow on CM2S agar medium containing 5mg/l of chloramphenicol and 25mg/ml of kanamycin were selected. The selected *C.glutamicum* YDK0101 stains containing pVSS1 and pPKSPTG11 were culture at 30°C for 3 days in a MMTG liquid medium (60g of glucose, 0.4g of magnesium sulfate heptahydrate, 30g of ammonium sulfate, 1g of potassium dihydrogen phosphate, 0.01g of iron sulfate heptahydrate, 0.01g of manganese sulfate pentahydrate, 450µg of thiamine hydrochloride, 450µg of biotin, 0.15g of DL-methionine, 50g of calcium carbonate, adjusted to 1L with water and pH 7.5) containing 5mg/l of chloramphenicol and 25mg/ml of kanamycin.

At the end of culture, 10 µl of the supernatant was subjected to SDS-PAGE. The purified transglutaminase secreted and produced by *C. glutamicum* ATCC113869 transformant was simultaneously electrophoresed as a standard Coomassie Brilliant Blue staining was conducted, which resulted in the detection of a band at the location of the same mobility as the standard. There were little other proteins found in the culture supernatant. The results confirmed that SMP45 was expressed and secreted normally and the pro-structural part of the secreted transglutaminases having pro-structure was cleaved and that a transglutaminase having the molecular weight which was almost identical to the naturally occurring mature transglutaminase was secreted.

### Example 1: Investigation on the added amount of pre-culture

Excess amount of the added pre-culture into the main culture would cause the reduction of the labeling index by stable isotopes. On the other hand, too small amount of the added pre-culture would decrease the growth of bacteria. Therefore, the optimum amount of the pre-culture which could be added into the main culture was tested.

The test was conduced with the kanamycin-resistant strain of *Corynebacterium glutamicum* UDK010 transformants, which was prepared in Reference Example 3 and into which the plasmid containing the EGF gene has been introduced. Firstly, the pre-culture was conducted for 16 hours in a CM2G liquid medium which was adjusted to contain 5g/L of glucose, 10g/L of polypeptone, 10g/L of yeast extract, 5g/L of NaCl, 0.2g/L of DL-methionine, pH7.2 and further containing 25mg/L of kanamycin.

Then a MMM liquid medium was adjusted to contain 60g/L of glucose, 30g/L of ammonium chloride, 0.15g/L of DL-methionine, 0.4g/L magnesium sulphate heptahydrate, 1g/L of potassium dihydrogen phosphate, 0.01g/L of iron sulfate heptahydrate, 0.01g/L of manganese sulfate pentahydrate, 450mg/L of thiamine hydrochloride, 450mg of biotin and 50g/L of calcium carbonate, pH7.5 and further containing 25mg/L of kanamycin.

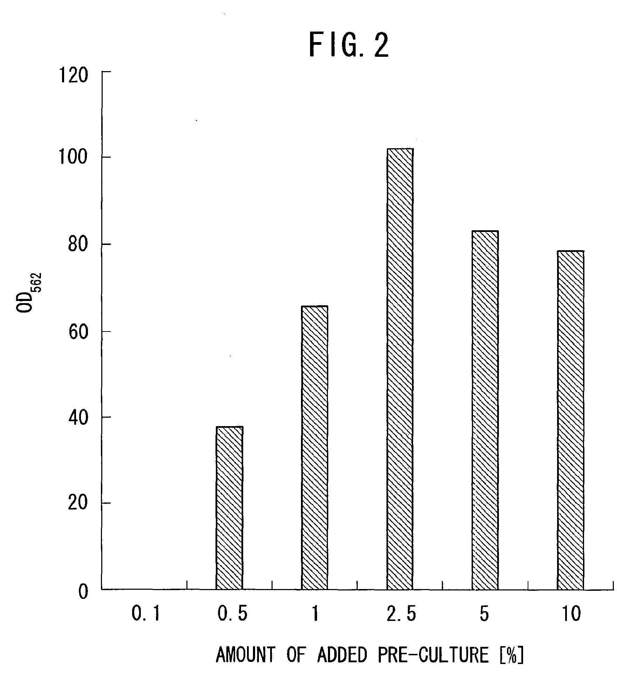

The amount to be incorporated was set to 0.1%, 0.5%, 1.0%, 2.5%, 5.0% and 10%, and the main culture was conducted at 30°C for 24 hours. The absorbance (OD562nm) during the main culture was shown in Figure 2.

It was shown that the preferable amount of the pre-culture which should be incorporated into the main culture was about 0.5% to about 10% and the optimum amount was about 2.5%, as indicated in Figure 2.

### Example 2: Labeling of EGF with stable isotopes by using coryneform bacteria

Proteins labeled with stable isotopes were prepared by using the kanamycin-resistant strain of *Corynebacterium glutamicum* UDK010 transformants, which was prepared in Reference Example 3. The CM2G liquid medium was prepared as described in Example 1 and the pre-culture was performed for 16 hours. The following experiments were conducted using ¹⁵N-labeled ammonium chloride(CIL) and ¹³C-glucose (CIL), having the labeling index of 99% and 99%, respectively.

### Preparation of the medium for ¹⁵N-labeling

¹⁵N-labeled MMM liquid medium was prepared to contain 60g/L of glucose, 30g/L of ¹⁵N-labeled ammonium chloride, 0.15g/L of 15N-labeled L-methionine, 0.4g/L of magnesium sulfate heptahydrate, 1g/L of potassium dihydrogen phosphate, 0.o1g/L of iron sulfate heptahydrate, manganese sulfate pentahydrate, 450mg/L of thiamine hydrochloride, 450mg of biotin and 50g/L of calcium carbonate, pH7.5 and further containing 25mg/L of kanamycin.

Another ¹⁵N-labeled MMM liquid medium was prepared, in which the amount to glucose and ¹⁵N-labeled ammonium chloride was changed to 40g/L or 17.5g, and 20g/L or 5g/L (100mM), respectively.

### Preparation of the medium for ¹³C- and ¹⁵N-labeling

¹³C-, ¹⁵N-labeled MMM liquid medium was prepared to contain 60g/L of ¹³C-glucose, 30g/L of ¹⁵N-labeled ammonium chloride, 0.15g/L of ¹⁵N-labeled L-methionine, 0.4g/L of magnesium sulfate heptahydrate, 1g/L of potassium dihydrogen phosphate, 0.o1g/L of iron sulfate heptahydrate, manganese sulfate pentahydrate, 450mg/L of thiamine hydrochloride, 450mg of biotin and 50g/L of calcium carbonate, pH7.5 and further containing 25mg/L of kanamycin.

Another ¹⁵N-labeled MMM liquid medium was prepared, in which the amount to glucose and ¹⁵N-labeled ammonium chloride was changed to 40g/L or 17.5g, and 20g/L or 5g/L (94mM), respectively.

The EGF producing strain was pre-cultured according to the similar procedure described in Example 1, and the pre-culture was transferred to a 500ml shaking flask containing 20m1 of ¹⁵N-labeled MMM liquid medium or ¹³C-, ¹⁵N-labeled MMM liquid medium at a ratio of 2.5% or 10% and was shaken for 24 hours at 30°C. As the control the culture was conducted with a similar medium containing unlabeled glucose and/or unlabeled ammonium chloride to calculate the labeling index for ¹⁵N-labeling and ¹³C-labeling. The EGFs prepared using ¹⁵N-labeled MMM liquid medium and ¹³C-, ¹⁵N-labeled MMM liquid medium were referred to as ¹⁵N-EGF and ¹³C-, ¹⁵N-EGF, respectively.

### Example 3: Calculation of labeling indices for ¹⁵N-EGF and ¹³C, ¹⁵N-EGF

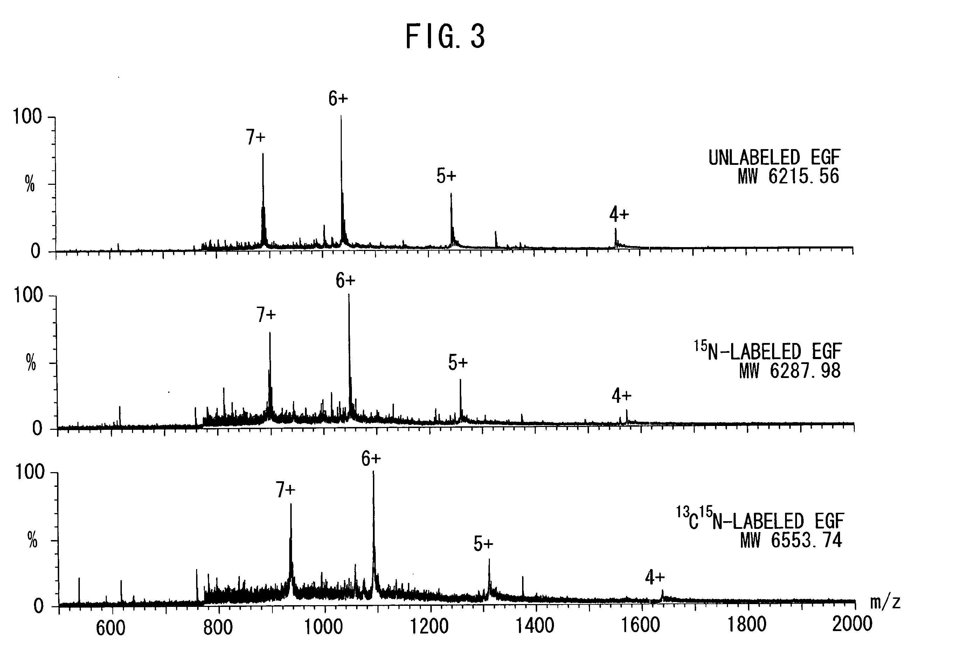

The culture supernatant of unlabeled EGF, ¹⁵N-EGF and ¹³C, ¹⁵N-EGF were purified by using HPLC column (Vydac, C18, 4.6mm diameter x 250mm length), a buffer containing 0.1% TFA and 0.1% TFA/90% acetonitrile, respectively, and the molecular weights determined by Q-TOF (Micromass) were sown in Figure 3. The concentration of glucose and ammonium chloride in the medium as a source of carbon and nitrogen, respectively, the amount of incorporated pre-culture and the labeling indices are summarized in Table 1.

**Table 1.**

| Concentration of glucose and ammonium chloride, the amount of incorporated pre-culture and the labeling indices | | |
|---|---|---|
| Amount of incorporated pre-culture 10% | | |
| Carbon and nitrogen source | | |
| | glucose 20g/L | glucose 60g/L |
| | ammonium chloride | ammonium chloride |
| | 5g/L(94mM) | 30g/L |
| ¹⁵N labeling index | 94.0% | 98.4% |
| | | |

| Amount of incorporated pre-culture 2.5% | | |
|---|---|---|
| Carbon and nitrogen source | | |
| | glucose 40g/L | glucose 60g/L |
| | ammonium chloride | ammonium chloride |
| | 17.5g/L | 30g/L |
| ¹⁵N labeling index | 99.7% | 99.6% |
| ¹³C labeling index | 99.2% | 98.8% |

The protein labeled with stable isotopes could be produced and secreted at an amount of about 2mg for EGF (about 9mg for MTG) by using 0.8g of glucose and 0.35g of ammonium chloride as the compounds labeled with stable isotopes in the case where the glucose concentration was 40g/L, the ammonium chloride concentration was 17.5g/L and the amount of incorporated pre-culture was at 2.5%.

The results of mass spectrometry revealed that the molecular weigh of EGF was 6215.6 and that the molecular weight of ¹⁵N-labeled EGF was 6288.0 and the molecular weight of ¹³C, ¹⁵N-labeled EGF was 6553.7 in the case where the pre-culture was added at an amount of 2.5%. Since the molecular weights would be 6288.5 and 6556.5, respectively, if the labeling indices of ¹⁵N-labeling and ¹³C-, ¹⁵N-labeling were 100%, which demonstrated that not less than 99% of labeling index was achieve for both of ¹⁵N-labeling and ¹³C-labeling.

### Example 4: Secretion and production of ²H-labeled EGF

Proteins labeled with ²H were prepared by using the kanamycin-resistant strain from *Corynebacterium glutamicum* UDK010 transformants, which was prepared in Reference Example 3. The medium was prepared with ²H₂O removing D, L-methionine from the ingredients of the medium for main culture described in Example 1 to confirm that the bacteria could grow in ²H₂O. The labeling index of ²H₂O was 98%. The EGF producing strain was pre-cultured similarly as described in Example 1, then 2ml of the pre-culture was added to 20ml of a main cultur medium prepared with ²H₂O and further cultured for 24 hours at 30°C.

As a result the absorbance (OD562nm) increased to 38.4 (determined by 100-fold dilution) which indicated that the bacteria grew well. The bacteria were further cultured for 48 hours and the supernatant was tested for EGF expression by SDS-PAGE as described in Example 3. The purification was conducted with the same HPLC as described in Example 3 and mass spectroscopy analysis was performed to calculate the labeling index by the stable isotopes. The results revealed the molecular weight of 6489.5, which demonstrated the labeling index of as high as 91%.

### Example 5: Production and secretion of labeled MTG

Proteins labeled with stable isotopes were prepared by using the kanamycin- and chloramphenicol-resistant strain from *Corynebacterium glutamicum* UDK010 transformants, which was prepared in Reference Example 3. CM2G medium was prepared as described in Example 1, and the bacteria were pre-cultured for 16 hours. The labeling indices of ¹⁵N-labeled ammonium chloride (CIL) and ¹⁵N-labeled L-methionine (Berlin Chemie) used were 99% and 95%, respectively, which were used for the experiments similar as Example 2.

### Example 6: NMR analysis of ¹⁵N-labeled EGF and MTG

Twenty (20) ml of the supernatant generated in Examples 2 and 5 was placed dialysis tubes having the pore size of molecular weight of 3500 (for EGF) or 6,000 (for MTG), which was repeatedly dialyzed against 20mM phosphate buffer, pH6 to remove low molecular weight molecules having the molecular weight of not more than 3500 or 6000. The supernatant was concentrated to 10-fold by using Centriprep (Millipore). NMR samples were prepared by adding 5% of ²H₂O for NMR lock.

Bruker DMX600 NMR Device equipped with triple nucleus triple gradient probes was used for NMR analysis. The temperature for measuring MTG and EGF was 35°C and 30°C, respectively. The resulting data were subjected to Fourier transformation using XWINNMR (Bruker). For HSQC spectra WATERGATE sequence using 3-9-19 pulse (J. Magn. Reson. Ser. A, 102, 241-245 (1993)) was inserted to suppress the water signal. The spectrum width was set to 10,000Hz for ¹H and 2,400Hz for ¹⁵N, and 1,024 points and 256 point were retrieved for t1 and t2 direction, respectively.

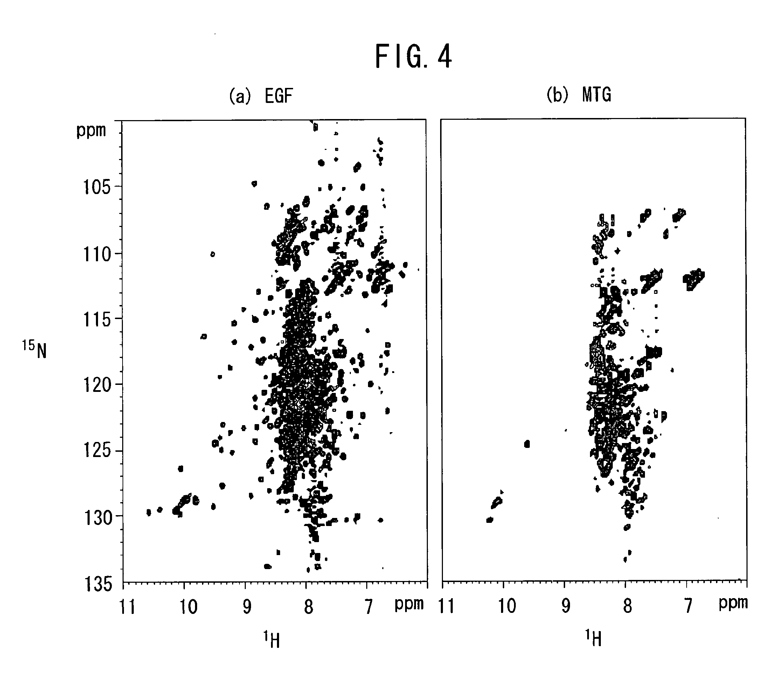

The results for EGF and MTG were shown in Figures 4a and 4b. As can be seen in Figures 4a and 4b, many signals from ¹H-¹⁵N were observed due to the incorporation of ¹⁵N-labeled ammonium chloride. Furthermore, the signals from EGF and MTG could be partially detected without purifying them.

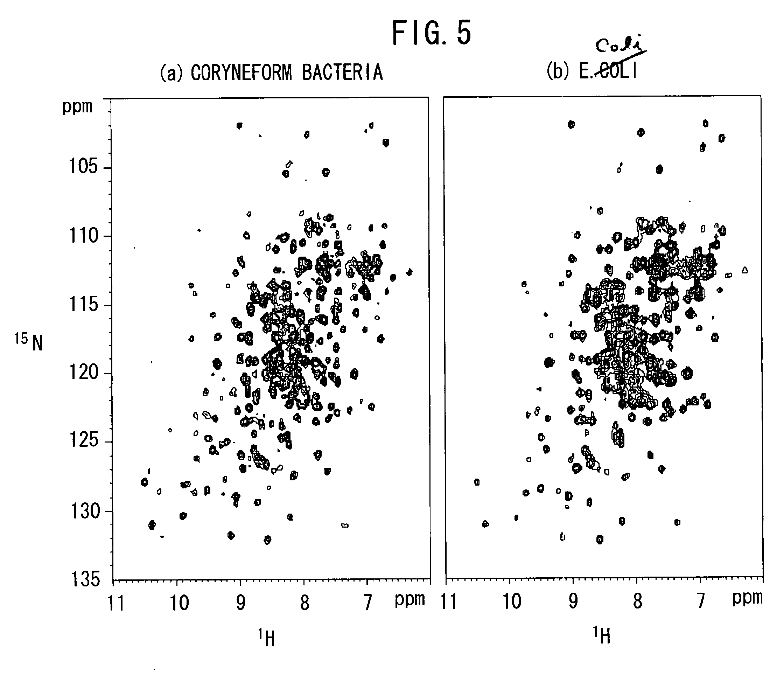

Additionally, the MTG producing strain was cultured at 20ml scale according to the similar procedure described in Example 5 and ¹⁵N-labeled MTG were purified by CM Sepharose. After repeated dialysis against 20mM phosphate buffer, pH6, the culture was concentrated to 700 µl by using Centriprep (Millipore) to which 5% of ²H₂O was added for NMR lock to prepare NMR samples. The resulting HSQC spectrum was shown in Figure 5a. The similar spectrum was determined for ¹⁵N-labeled MTG generated by E.coli (Figure 5b). These two spectra were almost identical, which demonstrate it was possible to prepare a sample which could be used for NMR analysis by coryneform bacteria.

The present invention provides a method of producing a protein labeled with a stable isotope conveniently at a low cost. In other words, according to the present invention, a protein labeled with a stable isotope and which has a high labeling index can be obtain in the active form by culturing a coryneform bacterium or *Bacillus brevis* at a high density in a medium containing labeled compounds at a high concentration, allowing the production of the protein of interest, and allowing the efficient secretion of the protein extracellularly.

Particularly, since coryneform bacteria natively secrete very little proteins extracellularly, the purifying steps of the protein according to the present invention, which was produced and secreted and which was labeled with a stable isotope, could be simplified or omitted, and the protein could be directly used for structural analysis such as NMR. Furthermore, the extracellular secretion of proteases is quite few, the secreted proteins with stable isotope-label would hardly undergo the degradation of by the proteases from the coryneform bacteria, which enable the efficient accumulation of the proteins labeled with stable isotopes in the culture solution. Additionally, coryneform bacteria and Bacillus bacteria can be cultured in a non-expensive complete synthetic medium containing ammonia or inorganic salts as its ingredients, therefore a protein having a high labeling index could be produced with non-expensive labeled compounds such as glucose or ammonium salts or the like. Furthermore, the amount of proteins derived form the medium is small in the culture solution, which make the purification much easier. Coryneform bacteria and Bacillus bacteria can be cultured in a medium containing sugars and ammonium salts at a high concentration, which also allow the production of stable isotopes having high labeling indices due to the increase of the concentration of stable isotopes in the medium. Additionally, the mass production of proteins which are labeled with stable isotopes and which have high labeling indices is possible by using a small amount of medium, because the bacteria can be cultured at a high density. Thus, the proteins can be produced at a low cost.

### References

1. Japanese Patent unexamined Published Application (JP Kokai) No. 6-502548.
2. JP Kokai No. 11-169182.
3. International Publication No. 01/23591 pamphlet.
4. JP Kokai No. 60-58074.
5. JP Kokai No. 62-201583.
6. JP Kokai No. 4-278091.
7. Mitsuhiko Ikura et al., "A novel approach for sequential assignment of proton, carbon-13, and nitrogen-15 spectra of larger proteins: heteronuclear triple-resonance three-dimensional NMR spectroscopy. Application to calmodulin", Biochemistry, 1990, vol. 29, p4659.
8. Jonathan Marley, Min Lu, and Clay Bracken, " A method for efficient isotopic labeling of recombinant proteins ", Journal of Biomol NMR, 2001, vol. 20, p71.
9. Harrold A. et al., " Efficient ¹³C/¹⁵ N double labeling of the avirulence protein AVR4 in a methanol-utilizing strain (Mut⁺) of Pichia pastoris", Journal of Biomol NMR, 2001, vol. 20, p251.
10. W. D. Morgan et al.," Expression of deuterium-isotope-labeled protein in the yeast Pichia pastoris for NMR studies", Journal of Biomol NMR, 2000, vol. 17, p337.
11. Kigawa T. et al., " Cell-free Synthesis and Amino Acid-selective Stable Isotope Labeling of Proteins for NMR Analysis", Journal of Biomol NMR, 1995, vol. 2, p129.
12. Yamagata H., " Use of *Bacillus brevis* for Efficient Synthesis and Secretion of Human Epidermal Growth Factor", Proc. Natl. Acad. Sci. USA, 1989, vol. 86, p3589.

## Claims

1. A method of producing a labeled protein with a stable isotpe, which comprises culturing a coryneform bacterium or a bacterium belonging to *Bacillus* in a medium which contains a carbon source and/or a nitrogen source labeled with one or more stable isotope selected from the group consisting of ²H, ¹³C and ¹⁵N and/or which contains ²H₂O, allowing the protein to be secreted into the medium and recovering the protein, wherein a genetic construct encoding the protein is introduced into the coryneform bacterium or the bacterium belonging to *Bacillus*.

2. The method of producing a protein labeled with a stable isotope according to claim 1, wherein the coryneform bacteria is *Corynebacterium glutamicum*.

3. The method of producing a protein labeled with a stable isotope according to claim 1, wherein the bacterium belonging to Bacillus is *Bacillus brevis*.

4. The method of producing a protein labeled with a stable isotope according to any one of claims 1 to 3, wherein the carbon source is selected from the group consisting of glucose, glycerol and sucrose.

5. The method of producing a protein labeled with a stable isotope according to any one of claims 1 to 4, wherein the nitrogen source is an ammonium salt.

6. The method of producing a protein labeled with a stable isotope according to any one of claims 1 to 4, wherein the labeled carbon source concentration is in the range of 5g/l to 150g/l calculated as glucose concentration and/or the labeled nitrogen source concentration is in the range of 30mM to 1M calculated as ¹⁵NH₄⁺ concentration.


SEQUENCE LISTING

<110> Ajinomoto Co., Inc.

<120> A method of producing stable isotope labeled proteins

<130> Y1K0690

<150> JP 2002-285254
<151> 2002-09-30

<160> 8

<170> PatentIn version 3.1

<210> 1
<211> 20
<212> DNA
<213> Artificial

<220>
<223> PCR primer

<400> 1
actgggaggc tatctccatt 20

<210> 2
<211> 20
<212> DNA
<213> Artificial

<220>
<223> PCR primer

<400> 2
atcgatctga tcacgttaca 20

<210> 3
<211> 40
<212> DNA
<213> Artificial

<220>
<223> PCR primer

<400> 3
tgtaacgtga tcagatcgat tcactggtcg acaccgttga 40

<210> 4
<211> 20
<212> DNA
<213> Artificial

<220>
<223> PCR primer

<400> 4
acggaagcta ccttcgaggt 20

<210> 5
<211> 37
<212> DNA
<213> Artificial

<220>
<223> PCR primer

<400> 5
gctcggtacc caaattcctg tgaattagct gatttag 37

<210> 6
<211> 24
<212> DNA
<213> Artificial

<220>
<223> PCR primer

<400> 6
gttgaagccg ttgttgatgt tgaa 24

<210> 7
<211> 42
<212> DNA
<213> Artificial

<220>
<223> PCR primer

<400> 7
aacatcaaca acggcttcaa caattccgat tctgagtgcc ct 42

<210> 8
<211> 20
<212> DNA
<213> Artificial

<220>
<223> PCR primer

<400> 8
cggccacgat gcgtccggcg 20